# EUROPEAN PATENT SPECIFICATION

(11) **EP 4 584 253 B1**
(45) Date of publication and mention of the grant of the patent: **11.11.2026**
(21) Application number: 23783093.0
(22) Date of filing: 06.09.2023
(51) Int. Cl.: C07D 333/36

(54) **PROCESS FOR THE PREPARATION OF DIMETHENAMID**
VERFAHREN ZUR HERSTELLUNG VON DIMETHENAMID
PROCÉDÉ DE PRÉPARATION DE DIMÉTHÉNAMIDE

(30) Priority: 07.09.2022 WO PCT/CN2022/117513
(43) Date of publication of application: 16.07.2025
(73) Proprietor: Adama Agan Ltd., 7710201 Ashdod (IL)
(72) Inventor: WANG, Kai, Beijing 101300 (CN); CHEN, Jianguo, Shanghai 201199 (CN); YACOVAN, Avihai, 7684704 Mazkeret Batya (IL); BAR NAHUM, Itsik, 7691000 Kfar-Gibton (IL)
(74) Representative: Modiano, Gabriella Diana
(86) International application number: PCT/IL2023/050959
(87) International publication number: WO 2024/052910

(56) References cited:
- EP-A1- 0 210 320
- EP-A2- 0 296 463
- WO-A1-95/21151
- CN-A- 113 024 505
- IL-A- 75 967
- US-A- 5 457 085
- BLASER* H-U ET AL: "Tunable ferrocenyl diphosphine ligands for the Ir-catalyzed enantioselective hydrogenation of N-aryl imines", JOURNAL OF ORGANOMETALLIC CHEMISTRY, ELSEVIER, AMSTERDAM, NL, vol. 621, no. 1-2, March 2001 (2001-03-01), pages 34 - 38, XP004231234, ISSN: 0022-328X, DOI: 10.1016/S0022-328X(00)00766-X

## Description

### FIELD OF THE INVENTION

The present invention relates to the field of synthesis of organic compounds, more specifically to a process for the preparation of Dimethenamid and intermediates thereof.

### BACKGROUND PRIOR ART

The agrochemical industry is always in the search of more efficient processes for the preparation of its active ingredients (Als). The capability of providing economical and clean synthesis of the active ingredients is one of the key factors determining the commercialization of an active ingredient.

Dimethenamid is a herbicide belonging to the group of chloroacetamides, that inhibits lipid synthesis. It is included in group 15 of the WSSA classification. It is typically applied on the soil to control a variety of broad-leaved weeds and grasses. It is a chiral molecule having two isomeric forms commonly known as M and P stereoisomers, dimethenamid-P being more biologically active.

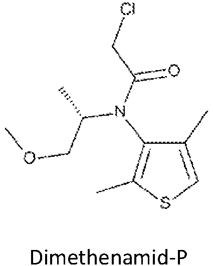

Synthetic schemes to prepare dimethenamid typically involve constructing the thiophene ring (typically a thiophen-3-one), followed by incorporating the 2-methoxy-2-propanamine (or 2-methoxyisopropylamine), also known as MOIPA, to finalize by coupling the 2-chloroacetamide moiety. One of the key steps is the incorporation of the MOIPA moiety, usually by condensation of 2,4-dimethyltetrahydrothiophen-3-one and/or 2,4-dimethyl-2,3-dihydrothiophen-3-one with S-2-methoxyisopropylamine (also known as S-MOIPA) to obtain N-(1-methoxyprop-2-yl)-2,4-dimethyl-3-aminothiophene.

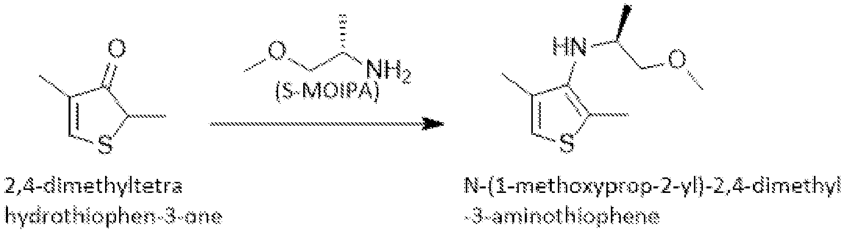

For example, EP 0 210 320 prepares the required 2,4-dimethyltetrahydrothiophen-3-one and then proceeds to react it with S-MOIPA in cyclohexene under heat and in the presence of molecular sieves (see example 1). The resulting imine is reacted under a number of different oxidation conditions to provide N-(1-methoxyprop-2-yl)-2,4-dimethyl-3-aminothiophene.

Later approaches seek the coupling of S-MOIPA to obtain N-(1-methoxyprop-2-yl)-2,4-dimethyl-3-aminothiophene in a single step. EP0296463 discloses in example 4 the reaction of a mixture of 2,4-dimethyl-2,3-dihydrothiophen-3-one and 2,4-dimethyl-3-hydroxythiophene and MOIPA in the presence of concentrated hydrochloric acid. The reaction directly provides N-(1-methoxyprop-2-yl)-2,4-dimethyl-3-aminothiophene after workup.

US 5,457,085 discloses a method for the preparation of N-(1-methoxyprop-2-yl)-2,4-dimethyl-3-aminothiophene by reacting 2,4-dimethyl-2,3-dihydrothiophen-3-one (compound of formula (II), can exist as a mixture with its tautomeric form 2,4-dimethyl-3-hydroxythiophenone) with S-MOIPA (compound of formula (III)). The reaction may use S-MOIPA as the solvent and a strong acid, such as hydrochloric acid, acetic acid, or trifluoroacetic acid. Example 7 discloses a process wherein S-MOIPA is used as solvent and concentrated hydrochloric acid is used in stochiometric amounts with respect to 2,4-dimethyl-3-thiophenone.

In CN113024505 the strategy is similar but, rather than S-MOIPA, it is the corresponding alcohol without the methyl group that it is used in the coupling with 2,4-dimethyl-3-thiophenone. The resulting product is then methylated to obtain N-(1-methoxyprop-2-yl)-2,4-dimethyl-3-aminothiophene.

None of these processes is completely satisfactory. There is therefore in the art a need to provide alternative procedures for obtaining dimethenamid and its intermediates. There is a need for processes that provide more efficient couplings between MOIPA and 2,4-dimethyl-3-thiophenone, providing for example higher yields, improved enantiomeric excesses, milder conditions and/or easier workups.

### SUMMARY OF THE INVENTION

The inventors have now realized that the election of the acid is key to obtaining good yields in the reaction to obtain N-(1-methoxyprop-2-yl)-2,4-dimethyl-3-aminothiophene.

Thus, a first aspect of the invention is a process to prepare N-(1-methoxyprop-2-yl)-2,4-dimethyl-3-aminothiophene or a salt thereof comprising contacting 2,4-dimethyl-2,3-dihydrothiophen-3-one with 2-methoxyisopropylamine, in the presence of one or more acid compounds selected from the group consisting of gaseous hydrochloric acid, an acid salt of 2-methoxyisopropylamine, a Lewis acid, triflic acid, and a mixture of hydrochloric acid with a C₁-C₆-alkylalcohol.

Typically, 2-methoxyisopropylamine is S-2-methoxyisopropylamine.

The process disclosed herein provides N-(1-methoxyprop-2-yl)-2,4-dimethyl-3-aminothiophene, salts in good to excellent yields and no (or very little) loss of enantiomeric excess. In order to arrive at dimethenamid (typically, dimethenamid-P) it is only necessary to add the 2-chloroacetyl moiety. It is therefore a further aspect a process for the preparation of dimethenamid-P that comprises
(i) preparing N-(1-methoxyprop-2-yl)-2,4-dimethyl-3-aminothiophene, salts thereof according to the process defined above; and
(ii) reacting the resulting N-(1-methoxyprop-2-yl)-2,4-dimethyl-3-aminothiophene, salts thereof, with 2-chloroacetyl chloride.

A further aspect**,** that is not part of the claimed invention, is the use of 2-methoxyisopropylamine to stabilize, store, pack or ship 2,4-dimethyl-2,3-dihydrothiophen-3-one.

A further aspect**,** that is not part of the claimed invention, is a method that comprises storing, packaging or shipping 2,4-dimethyl-2,3-dihydrothiophen-3-one in contact with 2-methoxyisopropylamine, a salt thereof. The inventors have observed that storing 2,4-dimethyl-2,3-dihydrothiophen-3-one overnight in the presence of air results in a loss of 10 to 20% of the material. On the other hand, no loss of material was observed when storing 2,4-dimethyl-2,3-dihydrothiophen-3-one in the presence of 2-methoxyisopropylamine under the same conditions.

Considering the literature, it was surprising that such acids provided a good yield and at the same time no loss of enantiomeric excess. For example, the use of concentrated hydrochloric acid reported in the literature produced a noticeable loss of enantiomeric excess. However, the use of gaseous hydrochloric acid or any of the above-mentioned acids prevented this problem.

An additional benefit observed was that the reaction proceeded at lower temperature. For example, a similar reaction in US 5,457,085 (example 7) requires more than 200°C in order to proceed, while the process of the invention proceeds to completion at temperatures as low as 120°C.

### DETAILED DESCRIPTION OF THE INVENTION

### Definitions

In the present document the following terms are given the meaning below.

Were indicated, the invention also provides salts of the compounds. For instance, salts of compounds provided herein may be acid addition salts, and they can be synthesized by conventional chemical methods from the parent compound which contains a basic moiety. Generally, such salts are, for example, prepared by reacting the free base forms of these compounds with a stoichiometric amount of the appropriate acid in water or in an organic solvent or in a mixture of both. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate.

The compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon, or ¹⁵N-enriched nitrogen, or ¹⁹F enriched fluorine are within the scope of this invention.

The starting material 2,4-dimethyl-2,3-dihydrothiophen-3-one also exists as its tautomeric form 2,4-dimethyl-3-hydroxythiophenone. The proportion between both tautomeric forms depends on different factors. For the purposes of the present application, they are both considered equivalent.

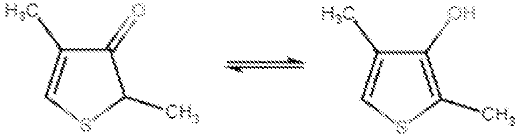

### The Acid

The process of the invention proceeds in the presence of one or more acids, preferably in catalytic amounts. The acid is one selected from the group consisting of the acid salt of 2-methoxyisopropylamine, a Lewis acid, triflic acid, gaseous hydrochloric acid and a mixture of hydrochloric acid with a C₁-C₆-alkylalcohol.

The reaction of 2,4-dimethyltetrahydrothiophen-3-one with MOIPA is known to proceed in the presence of concentrated hydrochloric acid (e.g. US 5,457,085). The inventors have found the use of concentrated hydrochloric acid unsatisfactory, mainly due to the loss of enantiomeric excess in the final product N-(1-methoxyprop-2-yl)-2,4-dimethyl-3-aminothiophene. It has been found that the use of an acid selected from the group consisting of the acid salt of 2-methoxyisopropylamine, a Lewis acid, triflic acid, gaseous hydrochloric acid and a mixture of hydrochloric acid with a C₁-C₆-alkylalcohol is surprisingly effective in preventing this problem.

For example, the acid salt of 2-methoxyisopropylamine, preferably used in catalytic amounts, completely prevents loss of enantiomeric excess under a number of conditions. An added benefit was that the reaction proceeds at lower temperature. Instead of adding an aqueous acid together with the 2-methoxyisopropylamine into the reaction, the corresponding salt of 2-methoxyisopropylamine is prepared prior to the reaction with 2,4-dimethyltetrahydrothiophen-3-one, and then added as any other reactant. Preferably, the preparation of the salt of 2-methoxyisopropylamine comprises contacting 2-methoxyisopropylamine with the corresponding acid in a solvent, preferably a non-aqueous solvent, followed by drying to remove excess water. The inventors have discovered that the reaction proceeds with better enantiomeric excess if the water content in the salt of 2-methoxyisopropylamine is 5% or less or less than 5%, as measured by Karl-Fischer titration. Any addition salt is suitable, for example a salt of 2-methoxyisopropylamine selected from the group consisting of HX, wherein X is a halide (e.g. hydrochloride, hydrobromide, hydroiodide), the sulphate, the nitrate, the phosphate, an organic acid addition salts such as, for example, acetate, maleate, fumarate, citrate, oxalate, succinate, tartrate, malate, mandelate, methanesulphonate or p-toluenesulphonate. It is preferred that the salt is HX, wherein X is a halide, for example, hydrochloride, hydrobromide or hydroiodide salts.

Thus, for example, the process disclosed herein comprises
a) providing an acid salt of 2-methoxyisopropylamine;
b) contacting 2,4-dimethyl-2,3-dihydrothiophen-3-one with said acid salt of 2-methoxyisopropylamine, in the presence of neutral 2-methoxyisopropylamine.

For example, the process disclosed herein comprises
a) providing an acid salt of 2-methoxyisopropylamine;
b) contacting 2,4-dimethyl-2,3-dihydrothiophen-3-one with at least one equivalent of 2-methoxyisopropylamine and a catalytic amount or stoichiometric amount of the acid salt of 2-methoxyisopropylamine.

Other acids useful for the process of the invention are Lewis acids. The inventors have discovered that a wide range of Lewis acids improve the yields of the reaction and at the same time prevent loss of enantiomeric excess. An added benefit of using Lewis acids is that the reaction proceeds at lower temperature. The inventors have tested a number of Lewis acids, such as Zn(OAc)₂, ZnCl₂, FeCl₂, FeCl₃, TiCl₄, Fe(OTf)₃ or BF₃·OEt₂ with good to excellent results. Thus, Lewis acids useful in the process disclosed herein are species capable of accepting a pair of electrons, such as the cations of some metals, for example cations of alkaline (e.g. Li⁺) and alkali earth (e.g. Mg²⁺) or cations of transition metals or rare-earth elements (e.g. Fe²⁺, Fe³⁺, Zn²⁺, Sc³⁺, Cu²⁺, Ti⁴⁺, Al³⁺, Sn²⁺, Sn⁴⁺, Ni²⁺, La³⁺, In³⁺, Ce³⁺ or Ce⁴⁺). Thus, Lewis acids useful in the process disclosed herein can be a cation of metals selected from the group consisting of Fe²⁺, Fe³⁺, Zn²⁺, Sc³⁺, Cu²⁺, Ti⁴⁺, Al³⁺, Sn²⁺, Sn⁴⁺, Ni²⁺, La³⁺, In³⁺, Ce³⁺, Ce⁴⁺, Li⁺ and Mg²⁺. Other Lewis acids are, for example, borane derivatives such as BF₃·OEt₂, or BCl₃ or others such as tributylphosphine, triphenylphosphine or TMSOTf (trimethylsilyltrifluoromethane sulfonate). Typically, the Lewis acid is the halide, acetate or -OTf salt of the above-mentioned cations. Thus, in the present invention, the Lewis acid is typically the halide, acetate or -OTf salt of a metal cation or a rare-earth element, for example chloride, acetate or -OTf. Non-limiting examples of Lewis acids useful in the process described herein is one selected from the group consisting of Zn(OAc)₂, ZnCl₂, ZnBr₂, FeCl₂, FeCl₃, TiCl₄, Fe(OTf)₃, BF₃·OEt₂, BCl₃, SnCl₄, Ni(OTf)₂ (OTf=trifluoromethanesulfonate), Zn(OTf)₂, Sc(OTf)₃, Sn(OTf)₂, Cu(OTf)₂, La(OTf)₃, In(OTf)₃, CeCl₃, CuCl₂, LiBr, Lil, LiCl, MgCl₂, MgBr₂, MgSO₄, AlCl₃, AlMeCl₂ and AlMe₂Cl. Typical examples according to the present disclosure are the halide or acetate salts of Zn(OAc)₂, Zn²⁺, Fe²⁺, Fe³⁺, Ti⁴⁺, Al³⁺ or Cu²⁺. A preferred Lewis acid is one selected from the group consisting of ZnCl₂, ZnBr₂, FeCl₂, FeCl₃, TiCl₄, Fe(OTf)₃, BF₃·OEt₂, SnCl₄, CuCl₂, and AlCl₃.

A further acid that has shown excellent results is triflic acid (trifluoromethanesulfonic acid or F₃C-S(O)₂OH). While aqueous concentrated hydrochloric acid resulted in a significant loss of enantiomeric excess in the product N-(1-methoxyprop-2-yl)-2,4-dimethyl-3-aminothiophene, gaseous hydrochloric acid provided excellent yields.

The acids can be added in catalytic or in stochiometric amounts or in excess. The acids are preferably added in catalytic amounts, typically in an amount of between 0.01 and 0.9 equivalents with respect to the 2,4-dimethyl-2,3-dihydrothiophen-3-one used as reactant. Typical catalytic amounts of acid are comprised between 0.01 and 0.5, for example between 0.02 and 0.4, preferably between 0.3 and 0.3 equivalents with respect to the 2,4-dimethyl-2,3-dihydrothiophen-3-one used as reactant.

The amount of S-MOIPA needed for the reaction to proceed to completion is 1 equivalent with respect to the 2,4-dimethyl-2,3-dihydrothiophen-3-one used as reactant, although an excess can be used to maximize conversion, for example from more than 1 equivalent to 10 equivalents, for example from more than one equivalent to 5 equivalents, or from 1.1 equivalents to 4 equivalents, or from 1.5 equivalents to 4 equivalents, for example, more than 1.2 equivalents or more than 1.5 equivalents or more than 1.8 equivalents with respect to the 2,4-dimethyl-2,3-dihydrothiophen-3-one starting material. The excess of S-MOIPA can be used advantageously so that it acts as solvent.

### The coupling of 2,4-dimethyl-2,3-dihydrothiophen-3-one with S-MOIPA

The reaction is preferably carried out at a temperature of from 50°C to 250°C; more preferably from 100°C to 250 °C; more preferably from 80°C to 230 °C; more preferably from 120°C to 200 °C, in particular from 110°C to 210 °C, for example from 150°C to 190 °C. For example, the reaction temperature is above 100°C, preferably above 110°C, more preferably above 120°C, more preferably above 150°C, more preferably above 160°C, more preferably above 170°C.

The reaction pressure is not critical. The temperatures used are sometimes higher than the boiling point of at least one of the solvents, and the reaction is then carried out in a closed vessel. This results in an inherent pressure above 1 bar, for example in the range of from 1.1 to 20 bar, in particular from 1.5 to 15 bar, for example from 3 to 12 bar. The reaction is preferably carried out in a pressure vessel, e.g. an autoclave.

The reaction can proceed either in the presence of a solvent or neat (without solvent). Preferred solvents are organic solvents, for example aliphatic hydrocarbons (e.g. hexane, heptane, cyclohexane, etc...) or aromatic hydrocarbons (e.g. toluene, xylene, etc.). Other organic solvents are also useful, for example ethers (e.g. diethyl ether, t-butylmethyl ether, diphenyl ether, etc...) or amides (e.g. dimethylformamide (DMF), dimethylacetamide (DMA) or N-methylpirryolidine (NMP)). In general, since the reaction typically takes place at a temperature between 50°C and 250°C, solvents (if used) preferably have a high boiling point, for example, above 90°C, for example above 100°C, typically between 90°C and 300°C or between 100°C and 250°C.

Therefore, it is possible to add a solvent to the reaction, although it is possible to add no solvent so that an excess of S-MOIPA is used and acts as solvent.

The time needed for the reaction can vary depending on many factors, such as the amount of acid added (the more acid, the faster the reaction), the temperature or the amount of S-MOIPA used. Considering these factors, the process disclosed herein can take from 1 minute to several hours or days, typical from one hour to 5 days, for example 48 hours, although typical times take from 2 hours to 36 hours, for example from 4 hours to 24 hours. The progress of the reaction can be followed using customary techniques (e.g. gas chromatography).

The reaction does not necessarily require special equipment and any vessel typically used for chemical reactions should be appropriate. In case some pressure is expected to build up, autoclave vessels or other measures to deal with pressure can be used.

The reaction is preferably carried out in an inert atmosphere to avoid the presence of oxygen, e.g. under an argon or nitrogen atmosphere.

The order in which the reagents are added is not critical. However, the inventors have discovered that neutral S-MOIPA has a surprising stabilizing effect over 2,4-dimethyl-2,3-dihydrothiophen-3-one. Therefore, it is preferable to add both together. It is even possible to create a stock solution of S-MOIPA and 2,4-dimethyl-2,3-dihydrothiophen-3-one which can be used in the reaction. It is thus another aspect of the invention a method that comprises storing, packaging or shipping 2,4-dimethyl-2,3-dihydrothiophen-3-one in contact with 2-methoxyisopropylamine, a salt thereof. The method thus comprises contacting 2,4-dimethyl-2,3-dihydrothiophen-3-one with 2-methoxyisopropylamine, a salt thereof without other reagents or solvents and storing both together for extended periods of time, for example for more than 1 hour, or more than 6 hours, or more than 12 hours, or more than 18 hours, or more than 24 hours, or more than 36 hours or more than 48 hours. The mixture stabilizes 2,4-dimethyl-2,3-dihydrothiophen-3-one and can be packaged and shipped without decomposition of 2,4-dimethyl-2,3-dihydrothiophen-3-one. It is thus a further aspect of the invention the use of 2-methoxyisopropylamine to stabilize, store, pack or ship 2,4-dimethyl-2,3-dihydrothiophen-3-one.

After completion of the reaction, the reaction mixture is worked up and N-(1-methoxyprop-2-yl)-2,4-dimethyl-3-aminothiophene, salts thereof is isolated in a customary manner. For example, the acid may be neutralized with a base (e.g. NaOH) to stop the reaction, and the solvents and/or excess S-MOIPA removed, for example under reduced pressure. If desired, the addition of a base may be avoided, and the workup proceed without the addition of a base, for example by removing the solvents and/or excess S-MOIPA, for example under reduced pressure, followed by the addition of water to remove any salts remaining. The resulting N-(1-methoxyprop-2-yl)-2,4-dimethyl-3-aminothiophene may be then purified following standard conditions (distillation, gel chromatography, recrystallization, etc ...).

It is possible to perform the method of the invention in different variants. Thus, for example, the process herein disclosed may comprise providing a mixture, preferably preformed, of 2,4-dimethyl-2,3-dihydrothiophen-3-one with MOIPA (preferably S-MOIPA), and putting them in contact with a preformed salt of S-methoxyisopropylamine S-MOIPA·HX (preferably in catalytic amounts with respect to the 2,4-dimethyl-2,3-dihydrothiophen-3-one), wherein X is chloride, bromide or fluor, the salt having less than a 5% content of water as measured by Karl-Fischer titration, preferably less than 3%. The salt is preferably the hydrochloric salt of S-MOIPA (S-MOIPA·HCl). The temperature is preferably comprised between 120°C and 200°C, and the reaction time is typically 6 to 36 hours.

For example, the process herein disclosed may comprise providing a mixture, preferably a preformed mixture, of 1 equivalent of 2,4-dimethyl-2,3-dihydrothiophen-3-one with 1 to 5 equivalents of MOIPA (preferably S-MOIPA), and putting them in contact with 0.01 to 1.0, for example with 0.01 to 0.9, preferably, 0.01 to 0.6, 0.05 to 0.5 or preferably 0.1 to 0.4 or 0.1 to 0.3, equivalents of a preformed salt of S-methoxyisopropylamine S-MOIPA·HX, wherein X is chloride, bromide or fluor, with respect to the 2,4-dimethyl-2,3-dihydrothiophen-3-one, the salt having less than a 5% content of water as measured by Karl-Fischer titration, preferably less than 3%. The salt is preferably the hydrochloric salt of S-MOIPA (S-MOIPA·HCl). The temperature is preferably comprised between 120°C and 200°C, and the reaction time is typically 6 to 36 hours.

For example, the process herein disclosed may comprise providing a mixture, preferably a preformed mixture, of 1 equivalent of 2,4-dimethyl-2,3-dihydrothiophen-3-one with 1 to 5 equivalents of MOIPA (preferably S-MOIPA), and putting them in contact with a solvent, preferably an aromatic hydrocarbon (e.g. toluene or xylene) or an ether (e.g. THF, MeTHF or diphenyl ether), and with 0.01 to 0.9 (preferably, 0.01 to 0.6, or preferably 0.01 to 0.4) equivalents of a preformed salt of S-methoxyisopropylamine S-MOIPA·HX, wherein X is chloride, bromide or fluor, with respect to the 2,4-dimethyl-2,3-dihydrothiophen-3-one, the salt having less than a 5% content of water as measured by Karl-Fischer titration, preferably less than 3%. The salt is preferably the hydrochloric salt of S-MOIPA (S-MOIPA·HCl). The temperature is preferably comprised between 120°C and 200°C, and the reaction time is typically 6 to 36 hours.

In another example, the process herein disclosed may comprise providing a mixture, preferably a preformed mixture, of 1 equivalent of 2,4-dimethyl-2,3-dihydrothiophen-3-one with 1.5 to 4 equivalents of MOIPA (preferably S-MOIPA), and putting them in contact with 0.05 to 0.6 equivalents of a preformed salt of S-methoxyisopropylamine S-MOIPA·HX, wherein X is chloride, bromide or fluor, with respect to the 2,4-dimethyl-2,3-dihydrothiophen-3-one, the salt having less than a 5% content of water as measured by Karl-Fischer titration, preferably less than 3%, and wherein no solvent is added. The salt is preferably the hydrochloric salt of S-MOIPA (S-MOIPA·HCl).

The use of the term "preformed mixture" refers to a mixture of 2,4-dimethyl-2,3-dihydrothiophen-3-one and 2-methoxyisopropylamine (preferably S-MOIPA) that has been prepared before the reaction. The mixture of both components can be prepared immediately before the reaction or it can be prepared before and stored, for example for more than 1 hour, or more than 6 hours, or more than 12 hours, or more than 18 hours, or more than 24 hours, or more than 36 hours or more than 48 hours.

A further example of the process disclosed herein comprises providing a mixture of 2,4-dimethyl-2,3-dihydrothiophen-3-one, MOIPA (preferably S-MOIPA) and a Lewis acid (preferably, a catalytic amount), optionally in the presence of a solvent. The temperature is preferably comprised between 120°C and 200°C, and the reaction time is typically 6 to 36 hours.

For example, the process may comprise providing a mixture of 2,4-dimethyl-2,3-dihydrothiophen-3-one, MOIPA (preferably S-MOIPA) and 0.01 to 0.9 equivalents (with respect to 2,4-dimethyl-2,3-dihydrothiophen-3-one), preferably, 0.01 to 0.6, or preferably 0.01 to 0.4, of a Lewis acid selected from the group consisting of Fe²⁺, Fe³⁺, Zn²⁺, Sc³⁺, Cu²⁺, Ti⁴⁺, Al³⁺, Sn²⁺, Sn⁴⁺, Ni²⁺, La³⁺, In³⁺, Ce³⁺, Ce⁴⁺, Li⁺, Mg²⁺, BF₃·OEt₂, BCl₃, tributylphosphine, triphenylphosphine and TMSOTf (trimethylsilyltrifluoromethane sulfonate). The temperature is preferably comprised between 120°C and 200°C, and the reaction time is typically 6 to 36 hours.

For example, the process may comprise providing a mixture of 1 equivalent of 2,4-dimethyl-2,3-dihydrothiophen-3-one, between 1 and 10, preferably between more than 1 and 4 equivalents of MOIPA (preferably S-MOIPA) and 0.01 to 0.9 equivalents (with respect to 2,4-dimethyl-2,3-dihydrothiophen-3-one), preferably, 0.01 to 0.6, or preferably 0.01 to 0.4, of a Lewis acid selected from the group consisting of Fe²⁺, Fe³⁺, Zn²⁺, Sc³⁺, Cu²⁺, Ti⁴⁺, Al³⁺, Sn²⁺, Sn⁴⁺, Ni²⁺, La³⁺, In³⁺, Ce³⁺, Ce⁴⁺, Li⁺, Mg²⁺, BF₃·OEt₂, BCl₃, tributylphosphine, triphenylphosphine and TMSOTf (trimethylsilyltrifluoromethane sulfonate). The temperature is preferably comprised between 120°C and 200°C, and the reaction time is typically 6 to 36 hours. The reaction is optionally performed in the presence of a solvent selected from the group consisting of aromatic hydrocarbons (e.g. toluene or xylene) and ethers (e.g. THF, MeTHF or diphenyl ether), although it can be performed without solvents (neat).

For example, the process may comprise providing a mixture of 1 equivalent of 2,4-dimethyl-2,3-dihydrothiophen-3-one, between 1 and 10, preferably between more than 1 and 4 equivalents of MOIPA (preferably S-MOIPA) and 0.01 to 0.4 equivalents (with respect to 2,4-dimethyl-2,3-dihydrothiophen-3-one) of a Lewis acid selected from the group consisting of Zn(OAc)₂, ZnCl₂, ZnBr₂, FeCl₂, FeCl₃, TiCl₄, Fe(OTf)₃, BF₃·OEt₂, BCl₃, SnCl₄, Ni(OTf)₂ (OTf=trifluoromethanesulfonate), Zn(OTf)₂, Sc(OTf)₃, Sn(OTf)₂, Cu(OTf)₂, La(OTf)₃ In(OTf)₃, CeCl₃, CuCl₂, LiBr, Lil, LiCl, MgCl₂, MgBr₂, MgSO₄, AlCl₃, AlMeCl₂ and AlMe₂Cl. The temperature is preferably comprised between 120°C and 200°C, and the reaction time is typically 6 to 36 hours. The reaction is optionally performed in the presence of a solvent selected from the group consisting of aromatic hydrocarbons (e.g. toluene or xylene) and ethers (e.g. THF, MeTHF or diphenyl ether), although it can be performed without solvents (neat).

For example, the process disclosed herein comprises providing a mixture of 2,4-dimethyl-2,3-dihydrothiophen-3-one, MOIPA (preferably S-MOIPA) and a catalytic amount of Zn²⁺, optionally in the presence of a solvent. The temperature is preferably comprised between 120°C and 200°C, and the reaction time is typically 6 to 36 hours.

For example, the process may comprise providing a mixture of 1 equivalent of 2,4-dimethyl-2,3-dihydrothiophen-3-one, between 1 and 10, preferably between more than 1 and 4 equivalents of MOIPA (preferably S-MOIPA) and 0.01 to 0.6 equivalents (with respect to 2,4-dimethyl-2,3-dihydrothiophen-3-one), preferably, 0.01 to 0.6, or preferably 0.01 to 0.4, of Zn²⁺ (for example, Zn(OAc)₂, ZnCl₂ or ZnBr₂). The temperature is preferably comprised between 120°C and 200°C, and the reaction time is typically 6 to 36 hours. The reaction is optionally performed in the presence of a solvent selected from the group consisting of aromatic hydrocarbons (e.g. toluene or xylene) and ethers (e.g. THF, MeTHF or diphenyl ether), although it can be performed without solvents (neat).

For example, the process may comprise providing a mixture of 1 equivalent of 2,4-dimethyl-2,3-dihydrothiophen-3-one, between 1 and 10, preferably between more than 1 and 4 equivalents of MOIPA (preferably S-MOIPA), between 0.5 and 5 equivalents of water (preferably 0.8 to 1.5), and 0.01 to 0.4 equivalents (with respect to 2,4-dimethyl-2,3-dihydrothiophen-3-one) of Zn²⁺ (for example, ZnCl₂ or ZnBr₂). The temperature is preferably comprised between 120°C and 200°C, and the reaction time is typically 6 to 36 hours. The reaction is optionally performed in the presence of a solvent selected from the group consisting of aromatic hydrocarbons (e.g. toluene or xylene) and ethers (e.g. THF, MeTHF or diphenyl ether), although it can be performed without solvents (neat).

For example, the process disclosed herein comprises providing a mixture of 2,4-dimethyl-2,3-dihydrothiophen-3-one, MOIPA (preferably S-MOIPA) and a catalytic amount of Fe²⁺ or Fe³⁺, optionally in the presence of a solvent. The temperature is preferably comprised between 120°C and 200°C, and the reaction time is typically 6 to 36 hours.

For example, the process may comprise providing a mixture of 1 equivalent of 2,4-dimethyl-2,3-dihydrothiophen-3-one, between 1 and 10, preferably between more than 1 and 4 equivalents of MOIPA (preferably S-MOIPA) and 0.01 to 0.9 equivalents (with respect to 2,4-dimethyl-2,3-dihydrothiophen-3-one), preferably, 0.01 to 0.6, or preferably 0.01 to 0.4, of Fe²⁺ or Fe³⁺ (for example, FeCl₂, FeCl₃, Fe(OTf)₃). The temperature is preferably comprised between 120°C and 200°C, and the reaction time is typically 6 to 36 hours. The reaction is optionally performed in the presence of a solvent selected from the group consisting of aromatic hydrocarbons (e.g. toluene or xylene) and ethers (e.g. THF, MeTHF or diphenyl ether), although it can be performed without solvents (neat).

For example, the process may comprise providing a mixture of 1 equivalent of 2,4-dimethyl-2,3-dihydrothiophen-3-one, between 1 and 10, preferably between more than 1 and 4 equivalents of MOIPA (preferably 2S-MOIPA) and 0.01 to 0.9 equivalents (with respect to 2,4-dimethyl-2,3-dihydrothiophen-3-one), preferably, 0.01 to 0.6, or preferably 0.01 to 0.4, of Ti⁴⁺ (for example, TiCl₄). The temperature is preferably comprised between 120°C and 200°C, and the reaction time is typically 6 to 36 hours. The reaction is optionally performed in the presence of a solvent selected from the group consisting of aromatic hydrocarbons (e.g. toluene or xylene) and ethers (e.g. THF, MeTHF or diphenyl ether), although it can be performed without solvents (neat).

For example, the process may comprise providing a mixture of 1 equivalent of 2,4-dimethyl-2,3-dihydrothiophen-3-one, between 1 and 10, preferably between more than 1 and 4 equivalents of MOIPA (preferably S-MOIPA) and 0.01 to 0.9 equivalents (with respect to 2,4-dimethyl-2,3-dihydrothiophen-3-one), preferably, 0.01 to 0.6, or preferably 0.01 to 0.4, of boron (for example, BF₃·OEt₂). The temperature is preferably comprised between 120°C and 200°C, and the reaction time is typically 6 to 36 hours. The reaction is optionally performed in the presence of a solvent selected from the group consisting of aromatic hydrocarbons (e.g. toluene or xylene) and ethers (e.g. THF, MeTHF or diphenyl ether), although it can be performed without solvents (neat).

For example, the process disclosed herein comprises providing a mixture of 2,4-dimethyl-2,3-dihydrothiophen-3-one, MOIPA (preferably S-MOIPA) and triflic acid, preferably in catalytic amounts, optionally in the presence of a solvent. The temperature is preferably comprised between 120°C and 200°C, and the reaction time is typically 6 to 36 hours.

For example, the process may comprise providing a mixture of 1 equivalent of 2,4-dimethyl-2,3-dihydrothiophen-3-one, between 1 and 10, preferably between more than 1 and 4 equivalents of MOIPA (preferably S-MOIPA) and 0.01 to 0.9 equivalents (with respect to 2,4-dimethyl-2,3-dihydrothiophen-3-one), preferably, 0.01 to 0.6, or preferably 0.01 to 0.4, of triflic acid. The temperature is preferably comprised between 120°C and 200°C, and the reaction time is typically 6 to 36 hours. The reaction is optionally performed in the presence of a solvent selected from the group consisting of aromatic hydrocarbons (e.g. toluene or xylene) and ethers (e.g. THF, MeTHF or diphenyl ether), although it can be performed without solvents (neat).

For example, the process disclosed herein comprises providing a mixture of 2,4-dimethyl-2,3-dihydrothiophen-3-one, MOIPA (preferably S-MOIPA) and gaseous hydrochloric acid (for example, in catalytic amounts), optionally in the presence of a solvent. The temperature is preferably comprised between 120°C and 200°C, and the reaction time is typically 6 to 36 hours.

For example, the process may comprise providing a mixture of 1 equivalent of 2,4-dimethyl-2,3-dihydrothiophen-3-one, between 1 and 10, preferably between more than 1 and 4 equivalents of MOIPA (preferably S-MOIPA) and 0.01 to 0.9 equivalents (with respect to 2,4-dimethyl-2,3-dihydrothiophen-3-one), preferably, 0.01 to 0.6, or preferably 0.01 to 0.4, of gaseous hydrochloric acid. The temperature is preferably comprised between 120°C and 200°C, and the reaction time is typically 6 to 36 hours. The reaction is optionally performed in the presence of a solvent selected from the group consisting of aromatic hydrocarbons (e.g. toluene or xylene) and ethers (e.g. THF, MeTHF or diphenyl ether), although it can be performed without solvents (neat).

For example, the process disclosed herein comprises providing a mixture of 2,4-dimethyl-2,3-dihydrothiophen-3-one, MOIPA (preferably S-MOIPA) and a mixture of methanol and hydrochloric acid (preferably in catalytic amounts), optionally in the presence of a solvent. The temperature is preferably comprised between 120°C and 200°C, and the reaction time is typically 6 to 36 hours.

For example, the process may comprise providing a mixture of 1 equivalent of 2,4-dimethyl-2,3-dihydrothiophen-3-one, between 1 and 10, preferably between more than 1 and 4 equivalents of MOIPA (preferably S-MOIPA) and 0.01 to 0.9 equivalents (with respect to 2,4-dimethyl-2,3-dihydrothiophen-3-one), preferably, 0.01 to 0.6, or preferably 0.01 to 0.4, of hydrochloric acid in a mixture with methanol, preferably in the absence of water. The temperature is preferably comprised between 120°C and 200°C, and the reaction time is typically 6 to 36 hours. The reaction is optionally performed in the presence of a solvent selected from the group consisting of aromatic hydrocarbons (e.g. toluene or xylene) and ethers (e.g. THF, MeTHF or diphenyl ether), although it can be performed without solvents (neat).

The method of the invention can use more than one of the compounds selected from an acid salt of 2-methoxyisopropylamine, a Lewis acid, gaseous hydrochloric acid, triflic acid, and a mixture of hydrochloric acid with a C₁-C₆-alkylalcohol. For example, the method can comprise providing a mixture, preferably a preformed mixture, of 1 equivalent of 2,4-dimethyl-2,3-dihydrothiophen-3-one with 1 to 5 equivalents of MOIPA (preferably S-MOIPA), and putting them in contact with a preformed salt of S-methoxyisopropylamine S-MOIPA·HX, wherein X is chloride, bromide or fluor, the salt having less than a 5% content of water as measured by Karl-Fischer titration, preferably less than 3%, and with a catalytic amount of an acid selected from the group consisting of a Lewis acid, gaseous hydrochloric acid, triflic acid, and a mixture of hydrochloric acid with a C₁-C₆-alkylalcohol. The salt is preferably the hydrochloric salt of S-MOIPA (S-MOIPA·HCl). The temperature is preferably comprised between 120°C and 200°C, and the reaction time is typically 6 to 36 hours. The Lewis acid, gaseous hydrochloric acid, triflic acid, or the mixture of hydrochloric acid with a C₁-C₆-alkylalcohol, can be added in amounts of 0.01 to 0.9 (preferably, 0.01 to 0.6, or preferably 0.01 to 0.4) equivalents, with respect to the amount of 2,4-dimethyl-2,3-dihydrothiophen-3-one. The preformed salt of S-methoxyisopropylamine S-MOIPA·HX can be added in catalytic or in stoichiometric amounts.

Thus, the process may comprise contacting 2,4-dimethyl-2,3-dihydrothiophen-3-one with 2-methoxyisopropylamine, the acid salt of 2-methoxyisopropylamine and a Lewis acid.

Thus, the process may comprise contacting 2,4-dimethyl-2,3-dihydrothiophen-3-one with 2-methoxyisopropylamine, the acid salt of 2-methoxyisopropylamine and gaseous hydrochloric acid.

Thus, the process may comprise contacting 2,4-dimethyl-2,3-dihydrothiophen-3-one with 2-methoxyisopropylamine, the acid salt of 2-methoxyisopropylamine and triflic acid.

Thus, the process may comprise contacting 2,4-dimethyl-2,3-dihydrothiophen-3-one with 2-methoxyisopropylamine, the acid salt of 2-methoxyisopropylamine and a mixture of hydrochloric acid with a C₁-C₆-alkylalcohol.

For example, the process may comprise contacting 2,4-dimethyl-2,3-dihydrothiophen-3-one with at least one equivalent of 2-methoxyisopropylamine, a catalytic amount of the acid salt of 2-methoxyisopropylamine and a catalytic amount of a Lewis acid, wherein the equivalents are measured with respect to the amount of 2,4-dimethyl-2,3-dihydrothiophen-3-one.

For example, the process may comprise contacting 2,4-dimethyl-2,3-dihydrothiophen-3-one with 2-methoxyisopropylamine, at least one equivalent of the acid salt of 2-methoxyisopropylamine and a catalytic amount of a Lewis acid, wherein the equivalents are measured with respect to the amount of 2,4-dimethyl-2,3-dihydrothiophen-3-one.

Preferred Lewis acids to be mixed with the acid salt of 2-methoxyisopropylamine are those comprising a cation of a metal selected from the group consisting of Fe²⁺, Fe³⁺, Zn²⁺, Sc³⁺, Cu²⁺, Ti⁴⁺, Al³⁺, Sn²⁺, Sn⁴⁺, Ni²⁺, La³⁺, In³⁺, Ce³⁺, Ce⁴⁺, Li⁺ and Mg²⁺. Thus, an alternative process of the invention comprises contacting 2,4-dimethyl-2,3-dihydrothiophen-3-one with 2-methoxyisopropylamine, the acid salt of 2-methoxyisopropylamine and a Lewis acid, wherein the Lewis acid is a cation of a metal selected from the group consisting of Fe²⁺, Fe³⁺, Zn²⁺, Sc³⁺, Cu²⁺, Ti⁴⁺, Al³⁺, Sn²⁺, Sn⁴⁺, Ni²⁺, La³⁺, In³⁺, Ce³⁺, Ce⁴⁺, Li⁺ and Mg²⁺. For example, the process may comprise contacting 2,4-dimethyl-2,3-dihydrothiophen-3-one with 2-methoxyisopropylamine, the acid salt of 2-methoxyisopropylamine and catalytic amounts of a Lewis acid, wherein the Lewis acid is a cation of a metal selected from the group consisting of Fe²⁺, Fe³⁺, Zn²⁺, Sc³⁺, Cu²⁺, Ti⁴⁺, Al³⁺, Sn²⁺, Sn⁴⁺, Ni²⁺, La³⁺, In³⁺, Ce³⁺, Ce⁴⁺, Li⁺ and Mg²⁺, and wherein the equivalents are measured with respect to the amount of 2,4-dimethyl-2,3-dihydrothiophen-3-one. It is preferred that the Lewis acid is the halide or acetate salt of a metal selected from the group consisting of Zn²⁺, Fe²⁺, Fe³⁺, Ti⁴⁺, Al³⁺ and Cu²⁺, more preferably, Zn(OAc)₂ or ZnCl₂. The process may comprise 0.01 to 0.9 equivalents (with respect to 2,4-dimethyl-2,3-dihydrothiophen-3-one), preferably, 0.01 to 0.6, or preferably 0.01 to 0.4 of the Lewis acid.

### EXAMPLES

### Example 1: General procedure for the preparation of N-(1-methoxyprop-2-yl)-2,4-dimethyl-3-aminothiophene or a salt thereof and screening with different acids

2,4-dimethyl-2,3-dihydrothiophen-3-one (130g, 96%, 1 mol), S-MOIPA (187g, 95%, 2eq) and the acid indicated in Table 1 were charged into a 1L autoclave under nitrogen atmosphere. The mixture was heated at the inner temperature indicated in Table 1 for 6h. Then, it was cooled to room temperature, 80ml water added and then 180g 30% NaOH (1.5eq). The mixture was distilled at atmospheric temperature to remove the excess of S-MOIPA. When cooled to room temperature again, enough water was added to dissolve the precipitated NaCl (if any). The oil layer was separated as the final product (crude). Where solvent was added, one volume was used. "One volume" means that 1ml of solvent is added for each gram of 2,4-dimethyl-2,3-dihydrothiophen-3-one.

**Table 1: summary of experiments**

| | M128 (mmol) | S-MOIPA (mmol) | Acid (equiv.) | T (°C) | Solvent | Yield % (ee%) |
|---|---|---|---|---|---|---|
| No acid (Comp.) | 1 | 2 | - | 150 | Toluene | 18 |
| Formic (Comp.) | 1 | 2 | 0.1 | 7 | Neat | 7 |
| H₂SO₄ (Comp.) | 1 | 2 | 0.1 | 160 | Neat | 35 |
| H₂SO₄ (Comp.) | 1 | 2 | 0.4 | 160 | Neat | 12 |
| p-toluene sulfonic acid (Comp.) | 1 | 1 | 1 | 100 | | >2% |
| Conc.HCl (Comp.) | 1 | 2 | 1 | 190 | Neat | 83 (85) |
| S-MOIPA·HCl (99 ee)¹ | 1 | 2 | 1 | 190 | Neat | 85 (96) |
| S-MOIPA·HCl (99 ee)² | 1 | 1.5 | 0.1 | 190 | Neat | 78(97) |
| S-MOIPA·HCl (99 ee)³ (50 g scale) | 1 | 2 | 0.1 | 190 | Neat | 80 (98) |
| S-MOIPA·HCl (99 ee)⁴ (50 g scale) | 1 | 2 | 0.5 | 190 | Neat | 82 (98) |
| S-MOIPA·HCl (99 ee)⁵ (50 g scale) | 1 | 1.5 | 0.1 | 170 | Neat | 83 (98) |
| Triflic acid | 1 | 2 | 0.5 | 170 | Neat | 90 (99) |
| Gaseous HCl | 1 | 2 | 0.0.5 | 190 | Neat | 88 (98) |
| HCl / MeOH | 1 | 2 | 1 | 190 | Neat | 87 (98) |
| ZnCl₂ | 1 | 2 | 0.1 | 150 | Neat | 60 |
| FeCl₂ | 1 | 2 | 0.1 | 160 | Neat | 76 (99) |
| FeCl₃ | 1 | 2 | 0.1 | 160 | Neat | 75 (99) |
| Fe(OTf)₃ | 1 | 2 | 0.1 | 160 | Neat | 80 |
| BF₃·OEt₂ | 1 | 1 | 0.1 | 150 | Toluene | 77 |

| | | | | | | |
|---|---|---|---|---|---|---|
| ¹ The water content of the S-MOIPA·HCl as by measured by Karl-Fischer titration was 5.3%. ² The water content of the S-MOIPA·HCl as by measured by Karl-Fischer titration was 1.48%. ³ The water content of the S-MOIPA·HCl as by measured by Karl-Fischer titration was 1.52%. ⁴ The water content of the S-MOIPA·HCl as by measured by Karl-Fischer titration was 1.45%. ⁵ The water content of the S-MOIPA·HCl as by measured by Karl-Fischer titration was 1.25%. | | | | | | |

As it can be observed in Table 1, the comparative examples either provided poor yields or a significant loss of enantiomeric excess. For example, p-toluene sulfonic acid provided less than 2% yield. The use of concentrated HCl reported in literature provided a good yield (85%), but at the same time a significant loss of enantiomeric excess (85% ee). Some acids even provided lower yields than the experiment without acid. On the other hand, the use of the acids according to the method of the invention provided good to excellent yields and at the same time excellent enantiomeric excesses.

### Example 2: Use of solvent

Following the excellent results obtained in Example 1, different solvents were tested to check consistency of the reaction. The reaction conditions followed the same procedure described in example 1 and one volume of solvent as indicated in Table 2. "One volume" means that 1ml of solvent is added for each gram of 2,4-dimethyl-2,3-dihydrothiophen-3-one.

**Table 2: Solvent**

| Solvent (volume) | M128 (equiv.) | S-MOIPA (equiv.) | Acid (equiv.) | T (°C) | Yield or Conversion (%) |
|---|---|---|---|---|---|
| Toluene | 1 | 3 | FeCl₃ (0.3) | 160 | 80 |
| Diphenyl ether | 1 | 2 | FeCl₂ (0.1) | 150 | 83 |
| Xylene | 1 | 2 | FeCl₂ (0.1) | 150 | 86 |
| Toluene | 1 | 2 | FeCl₂ (0.1) | 160 | 85 |
| THF | 1 | 2 | ZnCl₂ (0.2) | 150 | 75 |

Thus, the reaction also proceeded with good or excellent conversion in the presence of different solvents, while preserving the enantiomeric excess of the 2-methoxyisopropylamino moiety (no loss observed).

### Example 3: Reactions using ZnCl₂

The same procedure was followed as in example 1, in a 2 g scale with respect to 2,4-dimethyl-2,3-dihydrothiophen-3-one, with the following changes. Only 1 equivalent of S-MOIPA was added, ZnCl₂ was added in THF (1.0 M, 0.1 equiv.) and one volume of toluene was used as a solvent. The progress of the reaction was monitored by GC (IPC-1,24 h). Observed product peak was 75% after 24 hours at 150°C. Again, "one volume" means that 1ml of solvent is added for each gram of 2,4-dimethyl-2,3-dihydrothiophen-3-one.

The same reaction was performed but using 2 equivalents of S-MOIPA and 1 equivalent of water. After 12 hours at 150°C, the product peak represented 75%, and had an enantiomeric excess of >99%.

### Example 4: reactions using iron halides

The same procedure of Example 1 was followed on a 2 gram or 20 gram scale, using slightly different conditions to obtain yields of 70-80% with a 99% ee. In all cases the yield was good to excellent and no loss of enantiomeric excess was observed.

Examples 1 to 4 prove that the reaction supports a wide range of Lewis acids under different conditions of temperature, solvent or proportions between the reagents.

### Example 5: reactions using triflic acid

The same procedure of Example 1 was followed on a 1 gram or 20 gram scale using triflic acid. In each case the amounts, reagents and solvents indicated in Table 3 below.

**Table 3: triflic acid**

| Acid | M128 (equiv.) | S-MOIPA (equiv.) | Acid (equiv.) | T (°C) | solvent | Yield% (ee%) |
|---|---|---|---|---|---|---|
| Triflic acid | 1 | 2 | 0.1 | 160 | Neat | 90(99) |
| Triflic acid | 1 | 2 | 0.05 | 160 | Neat | 86(99) |

In all cases the yield was good to excellent, and no loss of enantiomeric excess was observed.

### Example 6: reactions using gaseous HCl

The same procedure of Example 1 was followed on a 1 gram or 20 gram scale using gaseous HCl (HCl(g)). In each case the amounts, reagents and solvents indicated in Table 4 below.

**Table 4: gaseous HCl**

| Acid | M128 (equiv.) | S-MOIPA (equiv.) | Acid (equiv.) | T (°C) | solvent | Yield% (ee%) |
|---|---|---|---|---|---|---|
| HCl (g) | 1 | 2 | 0.5 | 160 | Neat | 80 |
| HCl (g) | 1 | 2 | 0.5 | 175 | Neat | 85 |

In all cases the yield was good to excellent, and no loss of enantiomeric excess was observed.

### Example 7: reactions using an acid salt of S-MOIPA and a Lewis acid

**Table 5**

| | g | mmol | eq |
|---|---|---|---|
| M128/S-MOIPA mixed solution | 40 | 146.87/2115.73 | 1/1.47 |
| S-MOIPA | 7.00 | 77.84 | 0.53 |
| S-MOIPA·HCl | 18.62 | 146.87 | 1 |
| Zn(AcO)₂ | 2.75 | 14.69 | 0.1 |
| 30% NaOH aq. | 29.37 | 220.31 | 1.5 |
| MTBE | 50mL*2 | | |
| NaCl aq. | 50mL | | |

Zn(AcO)₂, S-MOIPA·HCl, S-MOIPA and a preformed mixture of 2,4-dimethyl-2,3-dihydrothiophen-3-one with S-MOIPA in the amounts indicated in table 5 were added into a 100mL autoclave, that was then heated to 180°C for 15h. After cooling, the sample was taken for GC detection. The GC result showed that the content of 2,4-dimethyl-2,3-dihydrothiophen-3-one was below 3%, with respect to the initial amount added.

The reaction solution was transferred to a 250mL reaction bottle, and 29.37g 30% NaOH aq. was added and stirred for 30min. The mixture was extracted two times with 50mL of MTBE (methyl-*tert*-butyl ether) and the organic layers combined. The resulting organic layer was washed with saturated NaCl solution, and then separated. The organic layer was distilled under vacuum, and finally kept at 50-60°C and 30-50mbar for 30min to yield 41.15g of crude N-(1-methoxyprop-2-yl)-2,4-dimethyl-3-aminothiophene. ee value was 99.53%, the yield was 88.96%.

### Example 8: Workup without base wash

2,4-dimethyl-2,3-dihydrothiophen-3-one (30g, 96%), S-MOIPA (2eq) and triflic acid (0.05 equiv.) were charged into an autoclave under nitrogen atmosphere. The mixture was heated at the inner temperature 160° for 22h. The pressure observed during the reaction was 3-4 bar. Once the reaction was finished, the mixture was distilled at atmospheric pressure to remove the excess of S-MOIPA. When cooled to room temperature, enough water was added to dissolve the precipitated salts. The oil layer was separated as the final product (crude). The yield observed was 94 % with an enantiomeric excess of more than 99%.

## Claims

1. A process for the preparation of N-(1-methoxyprop-2-yl)-2,4-dimethyl-3-aminothiophene or a salt thereof, the method comprising contacting 2,4-dimethyl-2,3-dihydrothiophen-3-one with 2-methoxyisopropylamine, in the presence of one or more components selected from the group consisting of an acid salt of 2-methoxyisopropylamine, a Lewis acid, gaseous hydrochloric acid, triflic acid, and a mixture of hydrochloric acid with a C₁-C₆-alkylalcohol.

2. The process according to claim 1, comprising
a) providing an acid salt of 2-methoxyisopropylamine;
b) contacting 2,4-dimethyl-2,3-dihydrothiophen-3-one with said acid salt of 2-methoxyisopropylamine.

3. The process according to any of the previous claims, wherein the acid is added in catalytic amounts or stoichiometric amount with respect to 2,4-dimethyl-2,3-dihydrothiophen-3-one.

4. The process according to any of the previous claims, wherein 2-methoxyisopropylamine is S-2-methoxyisopropylamine.

5. The process according to claim 1, wherein the Lewis acid is a cation of a metal selected from the group consisting of Fe²⁺, Fe³⁺, Zn²⁺, Sc³⁺, Cu²⁺, Ti⁴⁺, Al³⁺, Sn²⁺, Sn⁴⁺, Ni²⁺, La³⁺, In³⁺, Ce³⁺, Ce⁴⁺, Li⁺ and Mg²⁺.

6. The process according to claim 5, wherein the Lewis acid is the halide, acetate or -OTf salt of a cation of a metal selected from the group consisting of Fe²⁺, Fe³⁺, Zn²⁺, Sc³⁺, Cu²⁺, Ti⁴⁺, Al³⁺, Sn²⁺, Sn⁴⁺, Ni²⁺, La³⁺, In³⁺, Ce³⁺, Ce⁴⁺, Li⁺ and Mg²⁺.

7. The process according to any of claims 5 or 6, wherein the Lewis acid is one selected from the group consisting of Zn(OAc)₂, ZnCl₂, ZnBr₂, FeCl₂, FeCl₃, TiCl₄, Fe(OTf)₃, BF₃·OEt₂, BCl₃, SnCl₄, Ni(OTf)₂ (OTf=trifluoromethanesulfonate), Zn(OTf)₂, Sc(OTf)₃, Sn(OTf)₂, Cu(OTf)₂, La(OTf)₃, In(OTf)₃, CeCl₃, CuCl₂, LiBr, LiI, LiCl, MgCl₂, MgBr₂, MgSO₄, AlCl₃, AlMeCl₂ and AlMe₂Cl.

8. The process according to any of claims 5 to 7, wherein the Lewis acid is the halide or acetate salt of a metal selected from the group consisting of Zn²⁺, Fe²⁺, Fe³⁺, Ti⁴⁺, Al³⁺ and Cu²⁺.

9. The process according to any of claims 5 to 8, wherein the Lewis acid is one selected from the group consisting of Zn(OAc)₂, ZnCl₂, ZnBr₂, FeCl₂, FeCl₃, TiCl₄, Fe(OTf)₃, BF₃·OEt₂, SnCl₄, CuCl₂, and AlCl₃.

10. The process according to claim 1, comprising contacting 2,4-dimethyl-2,3-dihydrothiophen-3-one with 2-methoxyisopropylamine, the acid salt of 2-methoxyisopropylamine and a Lewis acid, wherein the Lewis acid is a cation of a metal selected from the group consisting of Fe²⁺, Fe³⁺, Zn²⁺, Sc³⁺, Cu²⁺, Ti⁴⁺, Al³⁺, Sn²⁺, Sn⁴⁺, Ni²⁺, La³⁺, In³⁺, Ce³⁺, Ce⁴⁺, Li⁺ and Mg²⁺, wherein the Lewis acid is in catalytic amounts as measured with respect to the amount of 2,4-dimethyl-2,3-dihydrothiophen-3-one.

11. The process according to claim 10, wherein the Lewis acid is the halide or acetate salt of a metal selected from the group consisting of Zn²⁺, Fe²⁺, Fe³⁺, Ti⁴⁺, Al³⁺ and Cu²⁺.

12. The process according to claim 11, wherein the Lewis acid is one selected from the group consisting of Zn(OAc)₂ and ZnCl₂.

13. The process according to any of the previous claims, wherein 2,4-dimethyl-2,3-dihydrothiophen-3-one is added as a preformed mixture of 2,4-dimethyl-2,3-dihydrothiophen-3-one with 2-methoxyisopropylamine.

14. The process according to any of the previous claims, wherein the reaction mixture is heated to a temperature comprised between 30°C and 200°C.

15. A process for the preparation of dimethenamid-P that comprises
(i) preparing N-(1-methoxyprop-2-yl)-2,4-dimethyl-3-aminothiophene or a salt thereof according to the process defined in any of claims 1 to 14; and
(ii) reacting the resulting N-(1-methoxyprop-2-yl)-2,4-dimethyl-3-aminothiophene, salts thereof, with 2-chloroacetyl chloride.

## Patentansprüche

1. Ein Verfahren zur Herstellung von N-(1-Methoxyprop-2-yl)-2,4-dimethyl-3-aminothiophen oder einem Salz davon, wobei das Verfahren das In-Kontakt-Bringen von 2,4-Dimethyl-2,3-dihydrothiophen-3-on mit 2-Methoxyisopropylamin in Anwesenheit eines oder mehrerer Bestandteile umfasst, die gewählt sind aus der Gruppe bestehend aus einem sauren Salz von 2-Methoxyisopropylamin, einer Lewis-Säure, einem gasförmigen Chlorwasserstoff, Trifluormethansulfonsäure und einer Mischung aus Salzsäure und einem C₁-C₆-Alkylalkohol.

2. Das Verfahren gemäß Anspruch 1, das Folgendes umfasst:
a) das Herstellen eines sauren Salzes von 2-Methoxyisopropylamin;
b) das In-Kontakt-Bringen von 2,4-Dimethyl-2,3-dihydrothiophen-3-on mit dem sauren Salz von 2-Methoxyisopropylamin.

3. Das Verfahren gemäß einem beliebigen der obigen Ansprüche, wobei die Säure in katalytischen Mengen oder einer stöchiometrischen Menge, bezogen auf 2,4-Dimethyl-2,3-dihydrothiophen-3-on, hinzugegeben wird.

4. Das Verfahren gemäß einem beliebigen der obigen Ansprüche, wobei 2-Methoxyisopropylamin S-2-Methoxyisopropylamin ist.

5. Das Verfahren gemäß Anspruch 1, wobei die Lewis-Säure ein Kation eines Metalls ist, gewählt aus der Gruppe bestehend aus Fe²⁺, Fe³⁺, Zn²⁺, Sc³⁺, Cu²⁺, Ti⁴⁺, Al³⁺, Sn²⁺, Sn⁴⁺, Ni²⁺, La³⁺, In³⁺, Ce³⁺, Ce⁴⁺, Li⁺ und Mg²⁺.

6. Das Verfahren gemäß Anspruch 5, wobei die Lewis-Säure das Halogenid, Acetat oder -OTf-Salz eines Kations eines Metalls ist, gewählt aus der Gruppe bestehend aus Fe²⁺, Fe³⁺, Zn²⁺, Sc³⁺, Cu²⁺, Ti⁴⁺, Al³⁺, Sn²⁺, Sn⁴⁺, Ni²⁺, La³⁺, In³⁺, Ce³⁺, Ce⁴⁺, Li⁺ und Mg²⁺.

7. Das Verfahren gemäß einem beliebigen der Ansprüche 5 oder 6, wobei die Lewis-Säure gewählt ist aus der Gruppe bestehend aus Zn(OAc)₂, ZnCl₂, ZnBr₂, FeCl₂, FeCl₃, TiCl₄, Fe(OTf)₃, BF₃·OEt₂, BCl₃, SnCl₄, Ni(OTf)₂(OTf=Trifluormethansulfonat), Zn(OTf)₂, Sc(OTf)₃, Sn(OTf)₂, Cu(OTf)₂, La(OTf)₃, In(OTf)₃, CeCl₃, CuCl₂, LiBr, LiI, LiCl, MgCl₂, MgBr₂, MgSO₄, AlCl₃, AlMeCl₂ und AlMe₂Cl.

8. Das Verfahren gemäß einem beliebigen der Ansprüche 5 bis 7, wobei die Lewis-Säure das Halogenid oder Acetatsalz eines Metalls ist, das gewählt ist aus der Gruppe bestehend aus Zn²⁺, Fe²⁺, Fe³⁺, Ti⁴⁺, Al³⁺ und Cu²⁺.

9. Das Verfahren gemäß einem beliebigen der Ansprüche 5 bis 8, wobei die Lewis-Säure gewählt ist aus der Gruppe bestehend aus Zn(OAc)₂, ZnCl₂, ZnBr₂, FeCl₂, FeCl₃, TiCl₄, Fe(OTf)₃, BF₃·OEt₂, SnCl₄, CuCl₂ und AlCl₃.

10. Das Verfahren gemäß Anspruch 1, das das In-Kontakt-Bringen von 2,4-Dimethyl-2,3-dihydrothiophen-3-on mit 2-Methoxyisopropylamin, dem sauren Salz von 2-Methoxyisopropylamin und einer Lewis-Säure umfasst, wobei die Lewis-Säure ein Kation eines Metalls ist, das gewählt ist aus der Gruppe bestehend aus Fe²⁺, Fe³⁺, Zn²⁺, Sc³⁺, Cu²⁺, Ti⁴⁺, Al³⁺, Sn²⁺, Sn⁴⁺, Ni²⁺, La³⁺, In³⁺, Ce³⁺, Ce⁴⁺, Li⁺ und Mg²⁺, wobei die Lewis-Säure in katalytischen Mengen vorliegt, gemessen mit Bezug auf die Menge von 2,4-Dimethyl-2,3-dihydrothiophen-3-on.

11. Das Verfahren gemäß Anspruch 10, wobei die Lewis-Säure das Halogenid oder Acetatsalz eines Metalls ist, das gewählt ist aus der Gruppe bestehend aus Zn²⁺, Fe²⁺, Fe³⁺, Ti⁴⁺, Al³⁺ und Cu²⁺.

12. Das Verfahren gemäß Anspruch 11, wobei die Lewis-Säure gewählt ist aus der Gruppe bestehend aus Zn(OAc)₂ und ZnCl₂.

13. Das Verfahren gemäß einem beliebigen der obigen Ansprüche, wobei 2,4-Dimethyl-2,3-dihydrothiophen-3-on als vorgeformte Mischung von 2,4-Dimethyl-2,3-dihydrothiophen-3-on mit 2-Methoxyisopropylamin hinzugegeben wird.

14. Das Verfahren gemäß einem beliebigen der obigen Ansprüche, wobei die Reaktionsmischung auf eine Temperatur zwischen 30°C und 200°C erwärmt wird.

15. Ein Verfahren zur Herstellung von Dimethenamid-P, das Folgendes umfasst:
(i) das Herstellen von N-(1-Methoxyprop-2-yl)-2,4-dimethyl-3-aminothiophen oder einem Salz davon gemäß dem Verfahren, das in einem beliebigen der Ansprüche 1 bis 14 definiert ist; und
(ii) das Reagieren des resultierenden N-(1-Methoxyprop-2-yl)-2,4-dimethyl-3-aminothiophen, Salzen davon, mit 2-Chloracetylchlorid.

## Revendications

1. Procédé de préparation de N-(1-méthoxyprop-2-yl)-2,4-diméthyl-3-aminothiophène ou d'un sel de celui-ci, ledit procédé comprenant la mise en contact de 2,4-diméthyl-2,3-dihydrothiophène-3-one avec 2-méthoxyisopropylamine, en présence d'un ou plusieurs composants choisis parmi le groupe constitué d'un sel acide de 2-méthoxyisopropylamine, d'un acide de Lewis, d'acide chlorhydrique gazeux, d'acide triflique et d'un mélange d'acide chlorhydrique avec un C₁-C₆-alkyli alcool en.

2. Procédé selon la revendication 1, comprenant les étapes:
a) fournir un sel acide de 2-méthoxyisopropylamine;
b) mettre en contact 2,4-diméthyl-2,3-dihydrothiophène-3-one avec ledit sel acide de 2-méthoxyisopropylamine.

3. Procédé selon quelconque des revendications précédentes, où l'acide est ajouté en quantités catalytiques ou en quantité stœchiométrique par rapport à 2,4-diméthyl-2,3-dihydrothiophène-3-one.

4. Procédé selon quelconque des revendications précédentes, où 2-méthoxyisopropylamine est S-2-méthoxyisopropylamine.

5. Procédé selon la revendication 1, où l'acide de Lewis est un cation d'un métal choisi dans le groupe constitué par Fe²⁺, Fe³⁺, Zn²⁺, Sc³⁺, Cu²⁺, Ti⁴⁺, Al³⁺, Sn²⁺, Sn⁴⁺, Ni²⁺, La³⁺, In³⁺, Ce³⁺, Ce⁴⁺, Li⁺ et Mg²⁺.

6. Procédé selon la revendication 5, où l'acide de Lewis est l'halogénure, l'acétate ou le sel -OTf d'un cation d'un métal choisi dans le groupe constitué par Fe²⁺, Fe³⁺, Zn²⁺, Sc³⁺, Cu²⁺, Ti⁴⁺, Al³⁺, Sn²⁺, Sn⁴⁺, Ni²⁺, La³⁺, In³⁺, Ce³⁺, Ce⁴⁺, Li⁺ et Mg²⁺.

7. Procédé selon quelconque des revendications 5 ou 6, où l'acide de Lewis est choisi parmi le groupe constitué de Zn(OAc)₂, ZnCl₂, ZnBr₂, FeCl₂, FeCl₃, TiCl₄, Fe(OTf)₃, BF₃·OEt₂, BCl₃, SnCl₄, Ni(OTf)₂ (OTf = trifluorométhanesulfonate), Zn(OTf)₂, Sc(OTf)₃, Sn(OTf)₂, Cu(OTf)₂, La(OTf)₃, In(OTf)₃, CeCl₃, CuCl₂, LiBr, LiI, LiCl, MgCl₂, MgBr₂, MgSO₄, AlCl₃, AlMeCl₂ et AlMe₂Cl.

8. Procédé selon quelconque des revendications 5 à 7, où l'acide de Lewis est le sel halogénure ou acétate d'un métal choisi dans le groupe constitué par Zn²⁺, Fe²⁺, Fe³⁺, Ti⁴⁺, Al³⁺ et Cu²⁺.

9. Procédé selon quelconque des revendications 5 à 8, où l'acide de Lewis est choisi parmi le groupe constitué de Zn(OAc)₂, ZnCl₂, ZnBr₂, FeCl₂, FeCl₃, TiCl₄, Fe(OTf)₃, BF₃·OEt₂, SnCl₄, CuCl₂ et AlCl₃.

10. Procédé selon la revendication 1, comprenant la mise en contact de 2,4-diméthyl-2,3-dihydrothiophène-3-one avec 2-méthoxyisopropylamine, le sel acide de 2-méthoxyisopropylamine et un acide de Lewis, où l'acide de Lewis est un cation d'un métal choisi dans le groupe constitué par Fe²⁺, Fe³⁺, Zn²⁺, Sc³⁺, Cu²⁺, Ti⁴⁺, Al³⁺, Sn²⁺, Sn⁴⁺, Ni²⁺, La³⁺, In³⁺, Ce³⁺, Ce⁴⁺, Li⁺ et Mg²⁺, où l'acide de Lewis est présent en quantités catalytiques par rapport à la quantité de 2,4-diméthyl-2,3-dihydrothiophène-3-one.

11. Procédé selon la revendication 10, où l'acide de Lewis est le sel halogénure ou acétate d'un métal choisi dans le groupe constitué par Zn²⁺, Fe²⁺, Fe³⁺, Ti⁴⁺, Al³⁺ et Cu²⁺.

12. Procédé selon la revendication 11, où l'acide de Lewis est choisi dans le groupe constitué par le Zn(OAc)₂ et le ZnCl₂.

13. Procédé selon quelconque des revendications précédentes, où 2,4-diméthyl-2,3-dihydrothiophène-3-one est ajoutée sous forme d'un mélange préformé de 2,4-diméthyl-2,3-dihydrothiophène-3-one et de 2-méthoxyisopropylamine.

14. Procédé selon quelconque des revendications précédentes, où le mélange de réaction est chauffé à une température comprise entre 30 °C et 200 °C.

15. Procédé de préparation du diméthénamide-P, qui comprend
(i) la préparation du N-(1-méthoxyprop-2-yl)-2,4-diméthyl-3-aminothiophène ou d'un sel de celui-ci selon le procédé défini dans quelconque des revendications 1 à 14 ; et
(ii) faire réagir N-(1-méthoxyprop-2-yl)-2,4-diméthyl-3-aminothiophène ainsi obtenu, ses sels, avec du chlorure de 2-chloroacétyle.
